# EUROPÄISCHE PATENTSCHRIFT

(11) **EP 1 266 650 B1**
(45) Veröffentlichungstag und Bekanntmachung des Hinweises auf die Patenterteilung: **03.11.2004**
(21) Anmeldenummer: 02010823.9
(22) Anmeldetag: 15.05.2002
(51) Int. Cl.: A61K 7/11

(54) **Haarwachsprodukt mit reduzierter Dichte**
Lower density hairwax
Cire de densité réduite pour cheveux

(30) Priorität: 12.06.2001 DE 10128468
(43) Veröffentlichungstag der Anmeldung: 18.12.2002
(73) Patentinhaber: Wella Aktiengesellschaft, 64274 Darmstadt (DE)
(72) Erfinder: Krause, Thomas, 64297 Darmstadt (DE); Stein, Bernd, 63728 Hösbach (DE); Kalbfleisch, Axel, 64295 Darmstadt (DE); Brocks, Werner, 36151 Burghaun (DE); Franzke, Michael, 64380 Rossdorf (DE); Schulz, Thomas, 64285 Darmstadt (DE)

(56) Entgegenhaltungen:
- EP-A- 0 692 248
- EP-A- 1 197 201
- DE-C- 19 610 459
- GB-A- 459 583
- US-A- 2 804 421

## Beschreibung

Gegenstand der Erfindung ist ein festes Haarwachsprodukt, welches mindestens ein festes Wachs oder einen festen wachsartigen Stoff enthält und dessen Dichte durch Eintragen einen Gases dauerhaft auf kleiner oder gleich 0,9 g/ml reduziert wurde.

Haarwachse werden seit längerer Zeit zur Frisurerstellung eingesetzt und dienen zur Formdefinition mit Hilfe der Finger. Sie finden insbesondere Anwendung, um kurzes bis mittellanges Haar trendgerecht in Form zu bringen und der Frisur Halt, Stand und Festigung sowie Glanz zu verleihen. Auch lassen sich mit Haarwachsen Konturen und Texture in der Frisur erzeugen. Herkömmliche Haarwachse werden üblicherweise in Tiegeln angeboten und beruhen auf folgendem Wirkungsprinzip: Die Entnahme der Produktmasse erfolgt mit den Fingern. Das Wachs wird in den Handflächen verteilt und durch die Handwärme aufgeschmolzen oder zumindest stark erweicht. Durch die Erweichung oder Aufschmelzung wird die Einarbeitung des ansonsten zu festen Wachses in das Haar ermöglicht. Im erweichten oder mehr oder weniger flüssigen Zustand wird das Wachs in das Haar eingearbeitet. Im Haar kühlt es ab und erreicht wieder seine Ausgangskonsistenz. Dabei erhärtet es und die gestaltete Frisur erhält Stabilität und Halt sowie häufig einen leichten Wet-Look. Durch dieses Wirkprinzip sind den einsetzbaren Wachsen und dadurch bedingt den erzielbaren Produktleistungen der herkömmlichen Stylingwachsprodukte enge Grenzen gesetzt. Damit sich das Wachs gut dem Tiegel entnehmen, gut verreiben und gut in das Haar einarbeiten lässt, darf es bei der Entnahme mit der Hand nicht zu hart sein und der Schmelz- oder Erweichunggspunkt muss in der Nähe der Körpertemperatur liegen. Die maximale Schmelz- oder Erweichungstemperatur herkömmlicher Haarwachsprodukte ist deshalb auf maximal ca. 40°C begrenzt. Andererseits läßt sich mit derartigen, relativ weichen Wachsen nur eine mäßige Produktleistung hinsichtlich Stand der Haare, Halt und Volumen der Frisur erreichen. Ausserdem ist die Belastung der Haare vergleichsweise hoch. Mit einer härteren, höherschmelzenden Wachszusammensetzung liesse sich zwar eine bessere Festigung und ein besserer Halt bei geringerer Einsatzmenge und geringerer Belastung erzielen, aber je härter das Wachs ist, umso härter ist auch die Produktmasse und umso schlechter kann diese dem Tiegel entnommen, verarbeitet und in die Haare eingearbeitet werden.

Die Aufgabe bestand darin, ein Haarwachs zu entwickeln, welches einen verbesserten Stand bei verringerter Belastung der Haare bewirkt und gleichzeitig aber gegenüber herkömmlichen Haarwachsprodukten vergleichbar gute Anwendungseigenschaften hinsichtlich Verreibarkeit und Einarbeitbarkeit in das Haar aufweist.

Ein weiterer Nachteil herkömmlicher Tiegelwachs-Produkte ist, dass bei der Anwendung die Wachsmasse in der Regel mit den Fingern aus einem Tiegel entnommen werden muß, was zu Verunreinigungen des im Tiegel verbleibenden Restproduktes führen kann. Eine weitere Aufgabe bestand deshalb darin, eine Applikationsform für Haarwachse zu finden, bei denen eine Verunreinigung der Produktmasse vermieden wird.

Es wurde gefunden, dass sich durch Aufschäumen einer Wachsmasse mit Gehalt an harten, hochschmelzenden Wachsen durch Gase die auf den ersten Blick eigentlich unvereinbaren Anforderungen überraschenderweise verwirklichen lassen. Durch die Aufschäumung der Wachsmasse und das Einbringen von teilweise auch optisch gut wahrnehmbaren Gasblasen oder Poren wird erreicht, dass die harte Wachsmasse unter mechanischer Belastung leichter mit den Händen verreibbar ist, obwohl die Temperatur der Hand des Anwenders deutlich unter der charakteristischen Erweichungstemperatur der Wachsmasse liegt. Dies ist vermutlich auf zwei verschiedene physikalische Effekte zurückzuführen: Zum einen muss an der Grenzfläche von Wachsmasse und Hand aufgrund der geringeren Dichte und größeren spezifischen Oberfläche der Masse eine geringere Wärmemenge zugeführt werden, um einen Gleitfilm an der Grenzfläche zu erzeugen. Durch den Gleitfilm wird dem Anwender ermöglicht, die Masse in seinen Händen besser zu zerreiben und durch Scherung aufzuschmelzen. Da die Masse nicht allein durch die Handwärme geschmolzen werden kann, wird der fehlende Energiebetrag durch Scherung der Masse eingetragen. Die Masse ist geeignet, bereits bei geringen Scherbelastungen relativ große Energiebeträge aufzunehmen, wie an dem Temperaturverhalten deutlich wird. Bereits bei geringer Erhöhung der Scherung erwärmt sich die Masse um mehrere °C.

Nach der Applikation am Haar und der damit verbundenen Abkühlung und Verfestigung sind durch diese harten Wachse weit größere Möglichkeiten der Frisurerstellung und Frisurengestaltung gegeben, da diese Wachse eine steifere Bündelung der Haare erlauben. Dieser Aspekt ist für ein trendorientiertes Stylingprodukt von großer Bedeutung.

Ein weiterer Vorteil der erfindungsgemäßen Produkte ist eine verbesserte Lagerstabilität bei höheren Temperaturen. Der Wärmetransport ins Innere der Masse wird durch die Blasenstruktur verhindert, da der Schaum wie ein Isolationsmaterial wirkt. Dieses Verhalten ist vorteilhaft, falls es zu einer unsachgemäß hohen Lagertemperatur kommt, da es ein Aufschmelzen der gesamten Masse und ein Entweichen der Gasblasen verhindert.

Ein weiterer Vorteil erfindungsgemäßer Produkte ist die Ermöglichung von im Vergleich zu herkömmlichen, in Tiegeln abgefüllten Haarwachsen vollkommen andersartigen Darreichungsformen. Durch die Härte der Masse bei Raumtemperatur ist es möglich, das Haarwachsprodukt in Form von dreidimensionalen Formkörpern, z.B. Tafeln, Blöcken, Stangen etc. herzustellen, welche vorteilhafterweise vordefinierte Bruchstellen aufweisen. Dies ermöglicht es dem Anwender, das Wachs hygienisch einwandfrei zu portionieren und die für eine Anwendung ausreichende Menge von der Tafel oder der Stange abzubrechen. Der Vorteil dieser Art der Darreichungsform ist die Verringerung der Verunreinigungen, die bei der Entnahme von herkömmlichen Haarwachsen mit den Fingern aus einem Tiegel nicht ausgeschlossen werden kann.

Ein weiterer Vorteil der Aufschäumung ist die deutlich sichtbare Verbesserung der kosmetischen Anmutung und des Aussehens der Masse. Die Mitverwendung von hochschmelzenden Wachsen, insbesondere Naturwachsen wie z.B. Bienenwachs bringt üblicherweise eine gelblich bräunliche Eigenfarbe in die Formulierung, die häufig unerwünscht ist. Durch das Aufschäumen mit Gasblasen wird die Farbe stark aufgehellt und das Produkt erscheint deutlich weisser, heller und angenehmer.

Die Verfahren, die sich zur Aufschäumung von den hier beschriebenen Wachsmassen eignen, werden bereits in der Lebensmittelindustrie zur Aufschäumung von halbfesten und cremigen Milchprodukten wie Joghurt oder Quark und fetthaltigen Produkten wie Schokolade eingesetzt(siehe z.B. GB, 459,583, US 5,238,698). Dabei kann das eingetragene Gas die diskontinuierliche Phase oder die kontinuierliche Phase (siehe z.B. US 4,889,738) bilden. Diese bekannten Verfahren sind prinzipiell auch zur Herstellung der erfindungsgemäßen Haarwachsprodukte geeignet.

Gegenstand der Erfindung ist ein bei Raumtemperatur (20°C) festes Haarwachsprodukt bestehend aus oder enthaltend mindestens ein Wachs und/oder mindestens einen wachsartigen Stoff. Das Produkt weist dauerhaft, d.h. auch nach Lagerung von mindestens einer Woche bei 20°C, eine Dichte von kleiner oder gleich 0,9 g/ml, vorzugsweise von 0,4 bis 0,8 g/ml auf. Erfindungsgemäß sind auch feste Haarwachsprodukte, die hergestellt werden, indem in eine mindestens ein Wachs und/oder mindestens einen wachsartigen Stoff enthaltende Wachszusammensetzung ein Gas unter Bedingungen eingetragen wird, unter denen die Wachszusammensetzung in flüssiger Form oder in einem für die Aufschäumung hinreichend erweichten Zustand vorliegt. Die erfindungsgemäßen Haarwachsprodukte werden zur Erzeugung von Halt, Festigung oder Stand der menschlichen Frisur verwendet.

In den erfindungsgemäßen Mitteln sind die Massen mit Luft oder einem inerten Gas in einer solchen Menge aufgeschäumt, dass die Dichte des fertigen Produktes kleiner oder gleich 0,9 g/ml, insbesondere 0,2 bis 0,8 g/ml, besonders bevorzugt 0,4 bis 0,7 g/ml beträgt. Als Gase kommen für das Aufschäumen neben Luft insbesondere inerte Gase in Betracht, z.B. Stickstoff, Kohlendioxid, Stickoxide, Edelgase oder Gemische der genannten Gase. Bevorzugt wird aus Kostengründen Pressluft. Bei der Mitverwendung von oxidationsempfindlichen Inhaltsstoffen ist die Verwendung von inerten, sauerstoffreien Gasen wie Stickstoff oder Kohlendioxid bevorzugt.

Die erfindungsgemäßen Haarwachsprodukte zeichnen sich dadurch aus, dass sie eine weitgehend homoge Verteilung von Poren oder Gasbläschen in einer festen Wachsmatrix aufweisen. Diese Poren- bzw. Blasenstruktur und die damit verbundene reduzierte Dichte ist stabil, d.h. sie bleibt über einen Zeitraum von mindestens 1 Woche, vorzugsweise mindestens 1 Monat, besonders bevorzugt mindestens 6 Monaten bei Lagerung bei Raumtemperatur (20°C) erhalten. Die Poren und Blasen haben typischerweise eine Größe von zwischen 0,0001 und 5 mm, bevorzugt zwischen 0,01 und 1 mm, besonders bevorzugt von 0,1 bis 0,8 mm.

### Wachsstoffe

Im folgenden wird für die erfindungsgemäß geeigneten Wachse und wachsartigen Stoffe der einheitliche Begriff "Wachs" verwendet. "Wachs" ist die technologische Sammelbezeichnung für eine Reihe von natürlichen, teilsynthetischen oder synthetisch gewonnenen Stoffen, die gemeinsame physikalische und anwendungstechnische Eigenschaften haben. Der Begriff "Wachs" bezieht sich insbesondere auf die Definition für Wachse gemäß Ullmanns Encyklopädie der technischen Chemie, 4. Auflage, Band 24, Seite 3. Demnach sind Wachse bei 20°C knetbar, fest bis brüchig hart, grob- bis feinkristallin, durchscheinend bis opak, jedoch nicht glasartig, über 40°C (Schmelz- bzw. Tropfpunkt) ohne Zersetzung schmelzend, schon wenig oberhalb ihres Schmelzpunktes verhältnismäßig niedrigviskos (z.B. Schmelzviskosität <10000 mPa s bei 10°C oberhalb des Tropfpunkts), stark temperaturabhängig in Konsistenz und Löslichkeit und unter leichtem Druck polierbar.

Das erfindungsgemäße Haarwachsprodukt weist vor dem Schäumen bei 25°C eine Nadelpenetrationszahl (Maßeinheit 0,1 mm, Prüfgewicht 100 g, Prüfdauer 5 s, Prüftemperatur 25°C; nach DIN 51 579) von vorzugsweise größer oder gleich 10, besonders bevorzugt größer oder gleich 20 auf. Die Wachse sind vorzugsweise in einer Menge von 5 bis 80 Gew.%, besonders bevorzugt von 20 bis 60 Gew.%, besonders bevorzugt von 30 bis 50 Gew.% enthalten und weisen in ungeschäumtem Zustand eine Nadelpenetrationszahl auf, welche kleiner ist als diejenige des fertigen, geschäumten Produktes.

Als Wachs kann prinzipiell jedes im Stand der Technik bekannte Wachs eingesetzt werden. Hierzu zählen Naturwachse (z.B. Insekten-, Tier- und Pflanzenwachse), fossile Wachse (z.B. Erdöl, Braunkohlen-, Torfwachse oder Ozokerite), mineralische Wachse, synthetische Wachse (z.B. Fischer-Tropsch-Wachse, Polyolefinwachse wie Polyethylen oder Polybuten, Amidwachse) und teilsynthetische Wachse, mikrokristalline Wachse, makrokristalline Wachse, Kohlenwasserstoffwachse, insbesondere feste, höherschmelzende Paraffine, Petrolatum, Vaseline, Montanwachs, sauerstofffunktionalisierte Kohlenwasserstoffwachse (z.B. erhalten durch Oxidation von Kohlenwasserstoffen wie z.B. Polyolefinen oder Paraffin oder durch Copolymerisation von Ethylen mit sauerstoffhaltigen Comonomeren wie Acrylsäure oder Vinylacetat), Bienenwachs, Wollwachs und dessen Derivate wie z.B. Wollwachsalkohole, Candelillawachs, Carnaubawachs, Japanwachs, gehärtete Fette, Fettsäuren und Fettalkohole mit z.B. 10-22 C-Atomen, Fettsäureester, Fettalkoholester, Fettsäureglyceride und Mono- oder Diester der Formeln R¹-(C=O)OR², R¹- (C=O) O-R³=O (C=O) R¹ und R²O(C=O)-R³-(C=O)OR² wobei R¹ für eine C8- bis C22-Alkylgruppe, R² für eine C3- bis C22-Alkylgruppe und R³ für eine C2- bis C16-Alkylengruppe steht, sowie Polyglykolwachse und Silikonwachse. Die Wachse haben einen Erstarrungspunkt oberhalb Raumtemperatur (20°C), vorzugsweise oberhalb 40°C, besonders bevorzugt oberhalb 55 °C. Die Nadelpenetrationszahl (0,1 mm, 100 g, 5 s, 25°C; nach DIN 51 579) liegt vorzugsweise im Bereich von 2 bis 70, insbesondere von 3 bis 40. Vorzugsweise ist mindestens ein Wachs enthalten, welches eine Nadelpenetrationszahl kleiner 40, besonders bevorzugt kleiner 20 aufweist. Bevorzugt ist Carnaubawachs und ein Ceresin mit einer Nadelpenetrationszahl kleiner 20 sowie deren Gemisch.

Besonders vorteilhaft aufgrund einer verbesserten Auswaschbarkeit ist ein Gehalt an Polyglykolwachsen, insbesondere an wachsartigen Polyethylenglykolen, Polyglykolmonomethylethern und Propylenoxid-Ethylenoxid Copolymeren. Besonders bevorzugt sind Polyethylenglykole (PEG). In Abhängigkeit vom Molekulargewicht sind geeignete PEGs weiche Wachse (800-2000 g/mol) oder harte Wachse (> 2000 g/mol), deren Schmelzpunkt gegen ein Maximum von ca. 60°C geht. Die hochmolekularen PEGs sind bei Raumtemperatur (20-25°C) wachsartig fest. Für die erfindungsgemäßen Produkte beträgt das Molekulargewicht insbesondere 2500 bis 6000 g/mol, vorzugsweise 2700 bis 5000 g/mol. Polyethylenglykole besitzen die allgemeine Formel H(OCH₂CH₂)ₙOH. Geeignete hochmolekulare Polyethylenglykole sind solche mit n = 57 bis 113, vorzugsweise mit n = 61 bis 79. Geeignete Polyethylenglykole haben die INCI-Bezeichnungen PEG-60 (n = 60), PEG-75 (n = 75), PEG-90 (n = 90) und PEG-100 (n = 100). Besonders bevorzugt sind PEG-60 und PEG-75. Handelsprodukte weisen in der Regel eine Molekulargewichtsverteilung auf. Geeignete Handelsprodukte sind z.B. Polyglykol 3000 mit einem Molekulargewicht von 2700 bis 3000 oder Polyglykol 4000 der Firma Clariant mit einem Molekulargewicht von 3700 bis 4500. Hierbei ist das Polyglykol 4000 vorteilhafter als das Polyglykol 3000, da es dem Haar einen besseren Stand verleiht. Zusätzlich können aber auch weichwachsartige PEGs mit einem Molekulargewicht von 800 bis 2000 g/mol enthalten sein.

### Zusätzliche hydrophobe Öle

Zur weiteren Verbesserung der erfindungsgemäßen Produkte können zusätzlich hydrophobe Fette oder Öle enthalten sein, von denen nicht-flüchtige Öle bevorzugt sind. Die Einsatzmenge kann bis zu 5 Gew.%, vorzugsweise 0,1 bis 4 Gew.% betragen. Nicht-flüchtige, hydrophobe Öle haben einen Schmelzpunkt von unterhalb 25°C und einen Siedepunkt von über 250 °C, vorzugsweise über 300 °C. Hierfür kann prinzipiell jedes dem Fachmann allgemein bekannte Öl eingesetzt werden. In Frage kommen pflanzliche oder tierische Öle, Mineralöle, Silikonöle oder deren Mischungen. Geeignete Silikonöle sind Polydimethylsiloxane, phenylierte Silikone, Polyphenylmethylsiloxane, Phenyltrimethicone, Poly(C1-C20)-alkylsiloxane, Alkylmethylsiloxane. Geeignet sind weiterhin Kohlenwasserstofföle, z.B. Paraffin- oder Isoparaffinöle, Squalan, Öle aus Fettsäuren und Polyolen, insbesondere Triglyceride. Geeignete pflanzliche Öle sind z.B. Sonnenblumenöl, Kokosöl, Rizinusöl, Lanolinöl, Jojobaöl, Maisöl, Sojaöl. Besonders bevorzugt sind Kohlenwasserstofföle, insbesonders Mineralöle (Paraffinum liquidum) und flüssige Alkane mit 14 oder mehr C-Atomen.

### Zusätzliche Emulgatoren

In einer bevorzugten Ausführungsform enthält das erfindungsgemäße Wachsprodukt zusätzlich mindestens einen Emulgator, um die Wiederauswaschbarkeit der Zusammensetzung aus dem Haar zu verbessern. Die Emulgatoren sind vorzugsweise in einer Menge von 0,1 bis 25 Gew.%, insbesondere 0,5 bis 20 Gew.%, besonders bevorzugt von 3 bis 15 Gew.% enthalten. Bevorzugte Emulgatoren sind solche aus der Klasse der nichtionischen Tenside. Geeignet sind z.B.
- Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
- C12- bis C22-Fettsäuremono- und -diester von Anlagerungsprodukten von 1 bis 30 mol Ethylenoxid an Glycerin und
- Anlagerungsprodukte von 5 bis 60 mol Ethylenoxid an Rizinusöl oder an gehärtetes (hydriertes) Rizinusöl.
- Mono-, Di- und/oder Triester der Phosphorsäure mit Anlagerungsprodukten von 2 bis 30 mol Ethylenoxid an C8- bis C22-Fettalkohole.

In einer besonders bevorzugten Ausführungsform haben die Emulgatoren selber ebenfalls wachsartige Konsistenz und einen Tropfpunkt oberhalb 25°C.

### Sonstige Inhaltsstoffe

Zusätzlich zu den vorstehend genannten Inhaltsstoffen kann die Haarwachszusammensetzung weitere Zusatzstoffe enthalten:
- Lösungsmittel wie Wasser oder ein- oder mehrwertige C1- bis C4-Alkohole, insbesondere Ethanol, Propanol, Glycerine oder Glykole in einer Menge bis zu 10 Gew.%, vorzugsweise 0,1 bis 8 Gew.%
- Kosmetische Farbstoffe in einer Menge bis zu 6 Gew.%, vorzugsweise 0,1 bis 4 Gew.%, z.B. C.I. Pigment Red 4 (C.I. 12 085), C.I. Pigment Green (C.I. 74 260), und/oder C.I. Vat Blue 4 (C.I. 69 800)
- Perlglanzpigmente in einer Menge von bis zu 25 Gew.%, vorzugsweise 1 bis 20 Gew.%, z.B. solche auf Titandioxid/Mica-Basis
- Parfüm- und Duftstoffe in einer Menge von bis zu 2 Gew.%, vorzugsweise 0,01 bis 1 Gew.%
- Konservierungsmittel in einer Menge von bis zu 1 Gew.%, vorzugsweise 0,01 bis 0,5 Gew.%, insbesondere Parahydroxybenzoesäureester, Benzoesäure, Salicylsäure, Sorbinsäure, Mandelsäure, Polyhexamethylenbiguanid-hydrochlorid oder Isothiazolinonderivate, von denen sich die Sorbinsäure als besonders geeignet erwiesen hat
- Filmbildende Polymere wie z.B. Polyvinylpyrrolidon oder Vinylpyrrolidon/Vinylacetat Copolymere in einer Menge von bis zu 5 Gew.%, vorzugsweise 0,1 bis 4 Gew.%
- Haarpflegende Zusätze wie z.B. Betain in einer Menge von bis zu 5 Gew.%, vorzugsweise 0,01 bis 4 Gew.%.

### Herstellungsverfahren

Das erfindungsgemäße Mittel kann hergestellt werden, indem
(1) eine ein Wachs und/oder einen wachsartigen Stoff enthaltende feste Zusammensetzung vorgelegt wird,
(2) diese Zusammensetzung durch Energieeintrag wie beispielsweise Erwärmen und/oder Scheren in eine flüssige Form oder in einen für eine Aufschäumung hinreichend erweichten Zustand überführt wird und
(3) in diese flüssige oder erweichte Zusammensetzung Luft und/oder ein inertes Gas, z.B. Stickstoff in einer solchen Menge eingetragen wird, dass die Dichte des fertigen Produktes kleiner oder gleich 0,9 g/ml beträgt.

Vorteilhafterweise wird die Wachszusammensetzung zunächst aufgeschmolzen bei einer Temperatur, die deutlich über deren Schmelz- bzw. Tropfpunkt liegt und die höher ist als die Temperatur, bei der die nachfolgende Aufschäumung erfolgt. Das aufgeschmolzene Wachs wird unter Scherung in einem Rührwerk temperiert. Durch den durch die Scherung erfolgenden Energieeintrag bleibt die Masse auch dann noch fließ- und rührfähig, wenn die Temperatur unterhalb des Erstarrungspunktes abgesenkt wird. Daher erfolgt das Aufschäumen unter ständiger Scherung bei Temperaturen, die typischerweise zwischen 10°C oberhalb des Erstarrungspunktes und vorzugsweise bis zu 40°C unterhalb des Erstarrungspunktes der Wachsmasse liegen. In die flüssige oder erweichte Wachsmasse wird das Gas eingetragen. Hierbei kann das Gas mittels Anlegen eines Unterdrucks (Vakuums) am Rührkessel und/oder mittels eines Überdrucks in der Gaszuführleitung zugeführt werden, wobei die Zuführung mit einem Überdruck von z.B. 1,2 bis 8 bar, vorzugsweise 2 bis 5 bar bevorzugt ist, z.B. unter Verwendung von Pressluft. Durch Veränderung der Fördermenge, Drehzahl, Temperatur, Druck und zugeführter Gasmenge lassen sich Dichte, Konsistenz und Schaumblasengröße wie gewünscht einstellen. Die eingetragene Gasmenge wird so gewählt, dass sich für das fertige Produkt eine Dichte von maximal 0,9 g/ml, vorzugsweise von 0,2 bis 0,8 g/ml, besonders bevorzugt von 0,4 bis 0,7 g/ml ergibt. Die mittlere Schaumblasen- bzw. Porengröße liegt vorzugsweise bei 0,1 bis 2 mm.

Wenn die Gaszuführung unter Überdruck geschieht, wird durch den Druckunterschied zwischen dem in der Mischapparatur herrschenden Druck und dem bei der Abfüllung herrschenden Atmosphärendruck eine Ausdehnung des in die Wachsmasse eingebrachten Gases unter Ausbildung der gewünschten Blasen- und Porenstruktur bewirkt. Durch Abkühlung und damit verbundener Verfestigung der Wachsmasse wird die Blasenstruktur stabilisiert. Der gleiche Effekt kann erreicht werden, indem zunächst das Gas unter Normaldruck in die fluide Wachsmasse eingebracht wird. Durch Anlegen eines Unterdrucks dehnen sich die eingebrachten Gasbläschen aus. Durch Abkühlen wird die gebildete Blasenstruktur stabilisiert.

Die stabil aufgeschäumten Wachsprodukte werden idealerweise in noch fließ- oder extrudierfähigem Zustand in geeignete Verpackungen wie z.B. Tiegel oder in geeignete Portionierformkörper abgefüllt. Die Produkte können als dreidimensionaler Formkörper ausgebildet werden und z.B. als Blöcke, Stangen oder Tafeln geformt werden. Sie enthalten dann vorzugsweise in der Art von Schokoladentafeln vorgegebene Sollbruchstellen, z.B. in der Form von Einkerbungen. Dies ermöglicht dem Anwender das Abbrechen von für eine Anwendung ausreichenden Portionen, ohne dass die verbleibende Restmenge kontaminiert wird.

### Anwendung

Die Produkte werden angewendet, indem eine für eine Anwendung ausreichende Produktmenge dem Verpackungsmittel entnommen bzw. von dem dreidimensionalen Formkörper mit Sollbruchstellen abgebrochen wird. Durch Verreiben des Produktes in den Handflächen wird über Scherung und Handwärme genügend Energie zugeführt, um die Masse soweit zu erweichen, dass sie in das Haar eingearbeitet werden kann. Nach dem Einarbeiten in das Haar kristallisieren die Wachse, verfestigen sich und verleihen dem Haar einen hervorragenden Stand.

### Beispiele

### Beispiel 1: Geschäumtes Haarwachs auf Basis hydrophober Wachse

Es wurde eine Wachsmasse aus folgenden Bestandteilen hergestellt:

| | |
|---|---|
| 46,4 g | Paraffinum Perliquidum |
| 25,0 g | Ceresin |
| 10,0 g | Carnaubawachs |
| 10,0 g | Triceteareth-4 Phsophat |
| 5,0 g | Bienenwachs |
| 3,0 g | ethoxyliertes (EO 25) und hydriertes Rizinusöl |
| 0, 4 g | Parfüm |
| 0,2 g | Propylparaben |

Die Wachsmasse wurde bei ca. 60 °C aufgeschmolzen und mittels einer Pumpe durch einen Kratz-Schabe-Kühler gefördert, wobei die Masse auf ca. 30-40°C temperiert wurde. Die Masse wurde weiter mit einer Fördergeschwindigkeit von 15-20 l/h dem Mischkopf des dynamischen Schaumgenerators Top Mix (Hansa Industrie Mixer) zugeführt. Mit einem Überdruck von 1,5 bis 5 bar wurde Stickstoff zugeführt und mit der Masse im Mischkopf bei 200-300 U/min vermischt. Hierbei wurde extern gekühlt, um eine durch die Scherenergie verursachte Temperaturerhöhung zu kompensieren. Die Masse wurde über ein Ventil aus dem Mischer mit einer Temperatur von ca. 30°C und mit butterartiger Konsistenz herausgefördert. Durch die Entspannung auf Normaldruck vergrößerten sich die in die Masse eingebrachten Gasblasen. Nach vollständigem Abkühlen war die Masse fest und hatte eine Dichte von 0,6 bis 0,8 g/ml.

Das geschäumte Produkt wies gegenüber der ungeschäumten Wachsmasse eine erheblich verbesserte Verreibarkeit und Einarbeitbarkeit in das Haar auf. Die optische Erscheinung war aufgrund der erfolgten deutlichen Aufhellung wesentlich angenehmer.

### Beispiel 2: Geschäumtes Haarwachs auf Basis von PEG-Wachsen

Es wurde eine Wachsmasse aus folgenden Bestandteilen hergestellt:

| | |
|---|---|
| 27,6 g | PEG-4 |
| 23,3 g | Glycerin |
| 21,0 g | PEG-90 |
| 20,0 g | PEG-60 |
| 3,8 g | Wasser |
| 3,0 g | ethoxyliertes (EO 25) und hydriertes Rizinusöl |
| 1,0 g | Parafinum Perliquidum |
| 0,3 g | Parfüm |

Die Herstellung des geschäumten Produktes erfolgte wie bei Beispiel 1. Das geschäumte Produkt wies gegenüber der ungeschäumten Wachsmasse eine erheblich verbesserte Verreibarkeit und Einarbeitbarkeit in das Haar auf.

## Patentansprüche

1. Festes Haarwachsprodukt bestehend aus oder enthaltend mindestens ein Wachs und/oder mindestens einen wachsartigen Stoff, **dadurch gekennzeichnet, dass** das Produkt aus einer festen Wachsmatrix mit einer Poren- oder Blasenstruktur besteht und eine gegenüber einem Produkt ohne Poren- oder Blasenstruktur dauerhaft reduzierte Dichte von kleiner oder gleich 0,9 g/ml aufweist.

2. Festes Haarwachsprodukt, **dadurch gekennzeichnet, dass** es hergestellt ist, indem in eine wachsförmige Zusammensetzung ein Gas unter Bedingungen eingetragen wird, unter denen die wachsförmige Zusammensetzung in flüssiger Form oder in einem für die Aufschäumung hinreichend erweichten Zustand vorliegt.

3. Haarwachsprodukt nach Anspruch 2, **dadurch gekennzeichnet, dass** es hergestellt ist durch
(A) Vorlegen einer ein Wachs und/oder einen wachsartigen Stoff enthaltenden festen Zusammensetzung,
(B) Überführen dieser Zusammensetzung durch Energieeintrag wie beispielsweise Erwärmen und/oder Scheren in eine flüssige Form oder in einen für eine Aufschäumung hinreichend erweichten Zustand,
(C) Aufschlagen oder Aufschäumen der Zusammensetzung mit Luft und/oder einem inerten Gas in einer solchen Menge, dass die Dichte des fertigen Produktes kleiner oder gleich 0,9 g/ml beträgt.

4. Haarwachsprodukt nach einem der vorhergehenden Ansprüche, **dadurch gekennzeichnet, dass** das Wachs oder der wachsartige Stoff ausgewählt ist aus Stoffen, welche bei 25°C eine Nadelpenetrationszahl von kleiner 40 aufweisen.

5. Haarwachsprodukt nach einem der vorhergehenden Ansprüche, **dadurch gekennzeichnet, dass** das Wachs oder der wachsartige Stoff ausgewählt ist aus Naturwachsen, fossilen Wachsen, synthetischen Wachsen, teilsynthetischen Wachsen, Kohlenwasserstoffwachsen, sauerstofffunktionalisierten Kohlenwasserstoffwachsen, Polyglykolwachsen, Silikonwachsen, Fettsäuren, Fettalkoholen, Fettsäureestern, Fettalkoholestern, wobei die genannten Substanzen vorzugsweise mindestens 12 C-Atome aufweisen, Mono- oder Diestern der Formeln R¹-(C=O)OR², R¹- (C=O) O-R³-O (C=O) R¹ und R²O(C=O)-R³-COOR², wobei R¹ für eine C8- bis C22-Alkylgruppe, R² für eine C3- bis C22-Alkylgruppe und R³ für eine C2- bis C16-Alkylengruppe steht.

6. Haarwachsprodukt nach einem der vorhergehenden Ansprüche, **dadurch gekennzeichnet, dass** das Wachs oder der wachsartige Stoff in einer Menge von 5 bis 80 Gew.% vorliegt.

7. Haarwachsprodukt nach einem der vorhergehenden Ansprüche, **dadurch gekennzeichnet, dass** das Produkt zusätzlich einen Emulgator enthält.

8. Haarwachsprodukt nach Anspruch 7, **dadurch gekennzeichnet, dass** der Emulgator ausgewählt ist aus
a) Anlagerungsprodukten von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
b) C12- bis C22-Fettsäuremono- und -diestern von Anlagerungsprodukten von 1 bis 30 mol Ethylenoxid an Glycerin,
c) Anlagerungsprodukten von 5 bis 60 mol Ethylenoxid an Rizinusöl oder an gehärtetes Rizinusöl,
d) Mono-, Di- und/oder Triestern der Phosphorsäure mit Anlagerungsprodukten von 2 bis 30 mol Ethylenoxid an C8- bis C22-Fettalkohole
oder aus Gemischen dieser Emulgatoren.

9. Haarwachsprodukt nach Anspruch 7 oder 8, **dadurch gekennzeichnet, dass** der Emulgator in einer Menge von 3 bis 20 Gew.% enthalten ist.

10. Haarwachsprodukt nach einem der vorhergehenden Ansprüche, **dadurch gekennzeichnet, dass** es mit Poren oder Gasblasen durchsetzt ist, welche eine mittlere Größe von 0,0001 bis 5 mm aufweisen.

11. Haarwachsprodukt nach einem der vorhergehenden Ansprüche, **dadurch gekennzeichnet, dass** es mit einem Gas aufgeschäumt ist, welches ausgewählt ist aus Luft, Stickstoff, Kohlendioxid, Stickoxiden, Edelgasen oder einem Gemisch dieser Gase.

12. Verfahren zur Herstellung eines Haarwachsproduktes gemäß einem der vorhergehenden Ansprüche, **dadurch gekennzeichnet, dass**
(A) eine ein Wachs und/oder einen wachsartigen Stoff enthaltende feste Zusammensetzung vorgelegt wird,
(B) diese Zusammensetzung durch Energieeintrag wie beispielsweise Erwärmen und/oder Scheren in eine flüssige Form oder in einen für eine Aufschäumung hinreichend erweichten Zustand überführt wird und
(C) in diese flüssige oder erweichte Zusammensetzung ein Gas in einer solchen Menge eingetragen wird, dass die Dichte des fertigen Produktes kleiner oder gleich 0,9 g/ml beträgt.

13. Verwendung einer Wachszusammensetzung, welche dauerhaft eine Dichte von kleiner oder gleich 0,9 g/ml aufweist und mindestens ein Wachs oder einen wachsartigen Stoff enthält zur Erzeugung von Halt, Festigung oder Stand der menschlichen Frisur, wobei die Wachszusammensetzung aus einer festen Wachsmatrix mit einer Poren- oder Blasenstruktur besteht.

## Claims

1. Solid hair wax product consisting of or comprising at least one wax and/or at least one wax-like substance, **characterized in that** the product comprises a solid wax matrix having a pore or bubble structure and, compared with a product without a pore or bubble structure, has a permanently reduced density of less than or equal to 0.9 g/ml.

2. Solid hair wax product, **characterized in that** it is prepared by introducing a gas into a waxy composition under conditions under which the waxy composition is in the liquid form or in a state which is sufficiently softened for foaming.

3. Hair wax product according to Claim 2, **characterized in that** it is prepared by
(A) initially introducing a solid composition comprising a wax and/or a wax-like substance into the preparation vessel,
(B) converting this composition, by introduction of energy, such as, for example, by heating and/or application of shear forces, into a liquid form or into a state which is sufficiently softened for foaming,
(C) whipping or foaming the composition with air and/or an inert gas in an amount such that the density of the finished product is less than or equal to 0.9 g/ml.

4. Hair wax product according to one of the preceding claims, **characterized in that** the wax or the wax-like substance is chosen from substances which have a needle penetration number of less than 40 at 25ºC.

5. Hair wax product according to one of the preceding claims, **characterized in that** the wax or the wax-like substance is chosen from naturally occurring waxes, fossil waxes, synthetic waxes, semisynthetic waxes, hydrocarbon waxes, oxygen-functionalized hydrocarbon waxes, polyglycol waxes, silicone waxes, fatty acids, fatty alcohols, fatty acid esters, fatty alcohol esters, wherein the substances mentioned preferably contain at least 12 C atoms, mono- or diesters of the formulae R¹- (C=O) OR², R¹- (C=O) O-R³-O (C=O) R¹ and R²O(C=O)-R³-COOR², wherein R¹ represents a C8- to C22-alkyl group, R² represents C3- to C22-alkyl group and R³ represents a C2- to C16-alkyl group.

6. Hair wax product according to one of the preceding claims, **characterized in that** the wax or the wax-like substance is present in an amount of 5 to 80 wt%.

7. Hair wax product according to one of the preceding claims, **characterized in that** the product additionally comprises an emulsifier.

8. Hair wax product according to Claim 7, **characterized in that** the emulsifier is chosen from
a) addition products of 2 to 30 mol of ethylene oxide and/or 1 to 5 mol of propylene oxide on C8- to C22-fatty alcohols, on C12- to C22-fatty acids or on alkylphenols having 8 to 15 C atoms in the alkyl group,
b) C12- to C22-fatty acid mono- and diesters of addition products of 1 to 30 mol of ethylene oxide on glycerol,
c) addition products of 5 to 60 mol of ethylene oxide on castor oil or on hydrogenated castor oil,
d) mono-, di- and/or triesters of phosphoric acid with addition products of 2 to 30 mol of ethylene oxide on C8- to C22-fatty alcohols
or from mixtures of these emulsifiers.

9. Hair wax product according to Claim 7 or 8, **characterized in that** the emulsifier is present in an amount of 3 to 20 wt%.

10. Hair wax product according to one of the preceding claims, **characterized in that** it is interspersed with pores or gas bubbles which have an average size of 0.0001 to 5 mm.

11. Hair wax product according to one of the preceding claims, **characterized in that** it is foamed with a gas which is chosen from air, nitrogen, carbon dioxide, nitrogen oxides, noble gases or a mixture of these gases.

12. Process for the preparation of a hair wax product according to one of the preceding claims, **characterized in that**
(A) a solid composition comprising a wax and/or a wax-like substance is initially introduced into the preparation vessel,
(B) this composition is converted, by introduction of energy, such as, for example, by heating and/or application of shear forces, into a liquid form or into a state which is sufficiently softened for foaming and
(C) a gas is introduced into this liquid or softened composition in an amount such that the density of the finished product is less than or equal to 0.9 g/ml.

13. Use of a wax composition which permanently has a density of less than or equal to 0.9 g/ml and comprises at least one wax or one wax-like substance for generating hold, set or shape of a human hairstyle, wherein the wax composition comprises a solid wax matrix having a pore or bubble structure.

## Revendications

1. Produit de cire coiffante en pâte, consistant en ou contenant au moins une cire et/ou au moins une matière cireuse, **caractérisé en ce que** le produit est constitué d'une matrice de type cire solide ayant une structure poreuse ou à bulles et présente une densité, réduite par rapport à un produit sans structure poreuse ou à bulles, inférieure ou égale à 0,9 g/ml.

2. Produit de cire coiffante en pâte, **caractérisé en ce qu'**il est préparé par introduction d'un gaz dans une composition cireuse, dans des conditions dans lesquelles la composition cireuse se trouve sous forme liquide ou en un état suffisamment ramolli pour le moussage.

3. Produit de cire coiffante selon la revendication 2, **caractérisé en ce qu'**il est préparé par
(A) disposition au préalable d'une composition solide contenant une cire et/ou une matière cireuse,
(B) conversion de cette composition, par apport d'énergie comme par exemple chauffage et/ou cisaillement, en une forme liquide ou en un état suffisamment ramolli pour un moussage,
(C) battage ou moussage de la composition avec de l'air et/ou un gaz inerte, en une quantité telle que la densité du produit final est inférieure ou égale à 0,9 g/ml.

4. Produit de cire coiffante selon l'une quelconque des revendications précédentes, **caractérisé en ce que** la cire ou la matière cireuse est choisie parmi des matières qui présentent à 25°C un indice de pénétration d'aiguille inférieur à 40.

5. Produit de cire coiffante selon l'une quelconque des revendications précédentes, **caractérisé en ce que** la cire ou la matière cireuse est choisie parmi des cires naturelles, des cires fossiles, des cires synthétiques, des cires partiellement synthétiques, des cires hydrocarbonées, des cires hydrocarbonées fonctionnalisées avec de l'oxygène, des cires polyglycol, des cires silicone, des acides gras, des alcools gras, des esters d'acides gras, des esters d'alcools gras, les substances citées comportant de préférence au moins 12 atomes de carbone, des mono- ou diesters de formules R¹-(C=O) OR², R¹- (C=O) O-R³-O (C=O) R¹ et R²O (C=O) -R³-COOR², R¹ représentant un groupe alkyle en C₈-C₂₂, R² représentant un groupe alkyle en C₃-C₂₂ et R³ représentant un groupe alkylène en C₂-C₁₆.

6. Produit de cire coiffante selon l'une quelconque des revendications précédentes, **caractérisé en ce que** la cire ou la matière cireuse est présente en une quantité de 5 à 80 % en poids.

7. Produit de cire coiffante selon l'une quelconque des revendications précédentes, **caractérisé en ce que** le produit contient en outre un émulsifiant.

8. Produit de cire coiffante selon la revendication 7, **caractérisé en ce que** l'émulsifiant est choisi parmi
a) des produits de fixation par addition de 2 à 30 moles d'oxyde d'éthylène et/ou de 1 à 5 moles d'oxyde de propylène sur des alcools gras en C₈-C₂₂, sur des acides gras en C₁₂-C₂₂ ou sur des alkylphénols ayant de 8 à 15 atomes de carbone dans le groupe alkyle,
b) des mono- et diesters d'acides gras en C₁₂-C₂₂ avec des produits de fixation par addition de 1 à 30 moles d'oxyde d'éthylène sur le glycérol,
c) des produits de fixation par addition de 5 à 60 moles d'oxyde d'éthylène sur l'huile de ricin ou sur de l'huile de ricin hydrogénée,
d) des mono-, di- et/ou triesters de l'acide phosphorique avec des produits de fixation par addition de 2 à 30 moles d'oxyde d'éthylène sur des alcools gras en C₈-C₂₂,
ou des mélanges de ces émulsifiants.

9. Produit de cire coiffante selon la revendication 7 ou 8, **caractérisé en ce que** l'émulsifiant est contenu en une quantité de 3 à 20 % en poids.

10. Produit de cire coiffante selon l'une quelconque des revendications précédentes, **caractérisé en ce qu'**il est traversé par des pores ou des bulles de gaz qui ont une taille moyenne de 0,0001 à 5 mm.

11. Produit de cire coiffante selon l'une quelconque des revendications précédentes, **caractérisé en ce qu'**il est transformé en mousse avec un gaz qui est choisi parmi l'air, l'azote, le dioxyde de carbone, les oxydes d'azote, les gaz rares ou un mélange de ces gaz.

12. Procédé pour la préparation d'un produit de cire coiffante selon l'une quelconque des revendications précédentes, **caractérisé en ce que**
(A) on dispose au préalable une composition solide contenant une cire et/ou une matière cireuse,
(B) on convertit cette composition, par apport d'énergie comme par exemple chauffage et/ou cisaillement, en une forme liquide ou en un état suffisamment ramolli pour un moussage,
(C) on introduit dans cette composition liquide ou ramollie un gaz en une quantité telle que la densité du produit final est inférieure ou égale à 0,9 g/ml.

13. Utilisation d'une composition de cire, qui présente durablement une densité inférieure ou égale à 0,9 g/ml et contient au moins une cire ou une matière cireuse, pour l'obtention de tenue, fixage ou dressage de la coiffure humaine, la composition de cire consistant en une matrice de cire solide ayant une structure poreuse ou à bulles.
